(19) **Europäisches Patentamt**
**European Patent Office**
**Office européen des brevets**

(11) Veröffentlichungsnummer: **0 070 397**
**B1**

(12) # EUROPÄISCHE PATENTSCHRIFT

(45) Veröffentlichungstag der Patentschrift:
**10.04.85**

(21) Anmeldenummer: **82105397.2**

(22) Anmeldetag: **19.06.82**

(51) Int. Cl.⁴: $C\ 07\ D\ 295/00$, $C\ 07\ D\ 265/30$, $B\ 01\ J\ 23/74$

(54) **Verfahren und Katalysator zur Herstellung von cyclischen Iminen.**

(30) Priorität: **30.06.81 DE 3125662**

(43) Veröffentlichungstag der Anmeldung:
**26.01.83 Patentblatt 83/4**

(45) Bekanntmachung des Hinweises auf die Patenterteilung:
**10.04.85 Patentblatt 85/15**

(84) Benannte Vertragsstaaten:
**BE CH DE FR GB IT LI**

(56) Entgegenhaltungen:
**EP - A - 0 002 630
DE - A - 2 738 115
DE - A - 3 116 395
FR - A - 2 461 706**

(73) Patentinhaber: **BASF Aktiengesellschaft,
Carl-Bosch-Strasse 38, D-6700 Ludwigshafen (DE)**

(72) Erfinder: **Schroeder, Wolfgang, Dr., Seebacher
Strasse 51, D-6702 Bad Duerkheim (DE)**
Erfinder: **Lengsfeld, Wolfgang, Dr., Woogstrasse 46,
D-6703 Limburgerhof (DE)**
Erfinder: **Heilen, Gerd, Dr., Schifferstadter Strasse 1B,
D-6720 Speyer (DE)**
Erfinder: **Hertel, Otto, Dr., Koenigsbacher Strasse 68,
D-6700 Ludwigshafen (DE)**
Erfinder: **Boettger, Guenter, Dr., Langen Wingert 16 B,
D-6702 Bad Duerkheim (DE)**

## Beschreibung

Die vorliegende Erfindung betrifft ein neues Verfahren zur Herstellung von cyclischen Iminen und einen Katalysator zur Durchführung des Verfahrens.

Cyclische Imine, wie Pyrrolidin, Piperidin, Morpholin oder Azepin sind wertvolle Zwischenprodukte für vielfältige Anwendungen, beispielsweise für Pflanzenschutzmittel und Pharmazeutika.

Morpholin, das auch als Vulkanisierhilfsmittel verwendet wird, ist das mengenmässig bedeutendste Produkt aus der Gruppe der cyclischen Imine. Seine Herstellung steht deshalb im Vordergrund der folgenden Beschreibung.

Bei den technisch üblichen Herstellungsverfahren für Morpholin geht man entweder aus von Diethanolamin, aus dem durch Wasserabspaltung Morpholin gewonnen wird, oder von Diethylenglykol (Diglykol), aus dem durch Anlagerung von Ammoniak unter Austritt von Wasser zunächst Aminodiglykol entsteht, das unmittelbar unter weiterem Verlust von Wasser und Ringschluss zu Morpholin weiterreagiert. Ähnliche Verfahren führen ausgehend von Butandiol zu Pyrrolidin und von Hexandiol zu Azepin (Hexamethylenimin).

Diese Umsetzungen werden üblicherweise entweder an einem feinteiligen Katalysator in überschüssigem Diol vorgenommen, wobei die Gegenwart von Wasserstoff günstig sein kann oder der Katalysator wird fest angeordnet (Festbett). Gewöhnlich wird bei hohem Druck, im allgemeinen im Bereich von 70 bis 350 bar, gearbeitet, wobei im Falle der Festbettanordnung bei der Umsetzung von Diethylenglykol dieses in flüssiger Phase über den Katalysator geleitet wird.

Ebenfalls fest angeordnet wird der Katalysator, wenn die Reaktionspartner gasförmig über den Katalysator geleitet werden sollen. Die hierfür verwendeten Katalysatoren sind entweder reine Nickelkatalysatoren (JA 71 31 863; RA 175 512) oder sie enthalten neben Nickel andere Metalle (JA 74 032 699; JA 74 032 189; DE 27 58 769).

Der Nachteil von Nickelkatalysatoren liegt in der schlechten Iminausbeute. Sie beträgt ca. 50 bis 80%.

Es wurde nun gefunden, dass man cyclische Imine durch Umsetzung von Diolen mit Ammoniak unter hydrierenden Bedingungen und in Gegenwart eines Mischkatalysators auf der Grundlage von Kupfer auf Aluminiumoxid, in hohen Ausbeuten herstellen kann, wenn man die Aminierung in der Gasphase an einem Katalysator vornimmt, der neben Kupfer 2 bis 50 Gew.-%, bezogen auf Kupfer, an Nickel und/oder Kobalt enthält und dessen Träger Aluminiumoxid ist.

Der Katalysator wurde dadurch erhalten, dass die mit Alkalicarbonat fällbaren Salze des Kupfers, Nickels bzw. Kobalts und des Aluminiums in wässriger Lösung mit Alkalicarbonat gefällt und die Fällung in üblicher Weise geformt, getrocknet und mit Wasserstoff aktiviert wurde.

Der Katalysator enthält als aktive Komponenten überwiegend Kupfer und ausserdem Nickel und/oder Kobalt. Der Gehalt an diesen Metallen beträgt 10 bis 70 Gew.-%, wobei der Rest im wesentlichen aus Aluminiumoxid besteht und der Gehalt an Nickel und/oder Kobalt Werte von 2 bis 50 Gew.-%, bezogen auf die Menge an Kupfer, aufweist.

Mit zunehmendem Nickelanteil wird allerdings die Reaktion zunehmend unspezifisch, so entsteht beispielsweise aus Diethylenglykol in erheblicher Menge Methylglykol. Verwendet man hingegen einen nickelfreien Katalysator, so ist die Aktivität nicht ausreichend hoch. Anstelle von Nickel lässt sich auch Kobalt verwenden. Dies ist jedoch, nicht zuletzt aus wirtschaftlichen Gründen, weniger vorteilhaft.

Der Katalysator kann nach der allgemeinen Vorschrift der DE-PS 24 45 303 hergestellt werden, die insoweit einen Bestandteil der vorliegenden Beschreibung bilden soll.

Der Katalysator kann sowohl in Form von zylindrischen Tabletten, als Extrudat mit kreisförmigem oder auch anderem Querschnitt oder in anderen Formen verwendet werden, wenn er fest angeordnet werden soll. Für die Verwendung in einem Wirbelbett, das ebenfalls als verfahrenstechnische Ausbildung in Frage kommt, wäre der Katalysator in Form eines Pulvers anzuwenden.

Zur Reduktion der Oxide des Kupfers und Nickels zum Metall wird zweckmässig ein Stickstoff-Wasserstoff-Gemisch mit 10 Volumen-% Wasserstoff verwendet. Das Kupferoxid wird im Temperaturbereich 140 bis 180°C reduziert. Zur Reduktion des Nickeloxids wird die Temperatur bis auf 300°C erhöht.

Der Katalysator eignet sich auch für andere Synthesen aus dem Gebiet der aminierenden Hydrierung.

Die nach dem erfindungsgemässen Verfahren herstellbaren cyclischen Imine bestehen vorzugsweise aus 5 bis 7 Ringgliedern, z.B. Pyrrolidin, Piperidin, Azepin, Morpholin. Sie können auch durch mehrere niedere Alkylgruppen substituiert sein.

Für die Reaktion geeignete Diole sind beispielsweise Butandiol-1,4, Pentandiol-1,5, Hexandiol-1,6, Diethylenglykol.

Der für die Reaktion benötigte Druck liegt beim erfindungsgemässen Verfahren unter 100 bar, meist unter 30 bar. Die Verdampfungs- und die Reaktionstemperatur liegt zwischen 170 und 250°C. insbesondere zwischen 200 und 250°C. Die beste Ausbeute hängt naturgemäss vom jeweiligen Katalysator ab und stellt sich im allgemeinen in einem ziemlich engen Temperaturbereich ein.

Der gesamte effektive Gasstrom liegt z.B. zwischen 100 und 1000 Volumenteilen je Volumenteil des Katalysators und Stunde. Er enthält z.B. zwischen 20 und 70 Volumen-% Ammoniak. Im allgemeinen ist es empfehlenswert, so wenig Ammoniak wie möglich anzuwenden.

Als Reaktionsapparate können Schachtöfen, bevorzugt jedoch Röhrenreaktoren verwendet werden. Die Strömungsrichtung ist vorzugsweise von oben nach unten.

Das Verfahren wird bevorzugt in folgender Weise durchgeführt: Mit Hilfe eines Kompressors wird ein im wesentlichen aus Wasserstoff bestehender Gasstrom aufrechterhalten, der nacheinander einen Verdampfer, den Reaktor, einen Kühler und einen Abscheider durchströmt und dann erneut vom Kompressor angesogen wird. Dem Verdampfer werden das Diol und Ammoniak zugeführt. Die Bedingungen im Verdampfer hinsichtlich Druck, Temperatur und Mengen sind so aufeinander abgestimmt, dass das Diol vollständig verdampft und ein Mengenverhältnis von Ammoniak zu Diol von z.B. 10 bis 100 Mol Ammoniak pro Mol Diol erhalten wird.

Die Reaktionstemperatur ist innerhalb gewisser Grenzen von dem Verhältnis Kupfer : Nickel im Katalysator abhängig. In allen Fällen findet man folgenden allgemeinen Zusammenhang: Unterhalb der günstigsten Temperatur enthält das rohe Reaktionsprodukt noch nicht umgesetztes Diol sowie reaktionsfähige Zwischenprodukte, mit fallender Temperatur steigt deren Anteil. Oberhalb dieser Temperatur enthält das rohe Reaktionsprodukt Spaltprodukte, bei Verwendung von Diethylenglykol vornehmlich Methylglykol, deren Anteil mit steigender Temperatur zunimmt. Der günstigste Bereich ist relativ eng, er umfasst etwa 10 bis 20° und liegt gewöhnlich zwischen 200 und 250°C. Die Einhaltung dieser Grenzen gelingt am einfachsten bei Verwendung eines Röhrenreaktors, der folglich bei der Durchführung des Verfahrens bevorzugt wird. Man legt die günstigste Temperatur, Strömungsgeschwindigkeit usw. durch orientierende Versuche fest.

Beim Kondensieren des Reaktionsproduktes wird ein Teil des nicht umgesetzten Ammoniaks gelöst. Die dem Verdampfer zugeführte Ammoniakmenge muss also sowohl diesen gelösten Teil auch auch den chemisch umgesetzten Teil ersetzen. Der gelöste Teil wird vom Reaktionsprodukt durch Destillation abgetrennt und dem Kreislaufgas wieder zugeführt. Das Reaktionsprodukt enthält im günstigen Falle im allgemeinen weniger als 2% nicht umgesetztes Diol, daneben bis zu 10% höher als das Imin siedende Zwischenprodukte, bei Verwendung von Diethylenglykol vor allem Morpholinyldiglykol, Morpholinyldiglykolamin, Dimorpholinyldiglykol. Eine Ausgestaltung des erfindungsgemässen Verfahrens besteht darin, diese leicht abtrennbaren Zwischenprodukte zusammen mit dem frischen Diol erneut dem Verdampfer und Reaktor zuzuführen, da sie letztlich in das Imin umgewandelt werden. Auf diese Weise wird eine Selektivität, bezogen auf das Diol, von etwa 95% erreicht.

*Beispiel 1*

### Katalysatorherstellung

Aus handelsüblichen Nitraten des Kupfers, Nickels und Aluminiums werden etwa 2molare wässrige Lösungen hergestellt und diese miteinander vereinigt. Dabei werden solche Mengen verwendet, dass die Metalle Kupfer, Nickel und Aluminium in einem Atomverhältnis 4 : 1 : 6 vorliegen.

Aus handelsüblichem Natriumcarbonat wird eine ebenfalls 2molare wässrige Lösung hergestellt.

Beide Lösungen werden auf 80°C gebracht und während 2 Stunden in einem Rührkessel so vereinigt, dass stets ein pH-Wert von 7 bis 8 aufrechterhalten wird. Es fällt ein Niederschlag aus, der abfiltriert und so lange mit salzfreiem Wasser gewaschen wird, bis er praktisch frei von Nitrat- und Natriumionen ist. Die erhaltene Katalysator-Rohmasse wird getrocknet, zu zylindrischen Tabletten von je 4,7 mm Höhe und Durchmesser verformt und diese bei 600°C getempert. Von diesem Katalysatorvorläufer wird 1 Liter in einen Rohrreaktor mit 45 mm Durchmesser gefüllt, der von einem wärmeträgerdurchflossenen Mantelrohr umgeben ist.

### Apparatur

Oberhalb des Reaktors befindet sich ein Verdampfer und eine Temperaturausgleichzone, in der das Gasgemisch auf Reaktionstemperatur gebracht werden kann.

Unterhalb des Reaktors schliesst sich ein Kühler an, in dem das Reaktionsgemisch auf etwa 20°C gekühlt werden kann und anschliessend ein Abscheider. Das nicht kondensierte Gas kann mit Hilfe eines Kompressors zum Verdampfer zurückgeführt werden, dem auch die übrigen Reaktionspartner zugeführt werden.

### Aktivierung des Katalysators

Vor Reaktionsbeginn wird der Katalysatorvorläufer durch Reduzieren mit Wasserstoff zum eigentlichen Katalysator umgewandelt. Es wird ein Gemisch aus 10 Vol.-% Wasserstoff und 90 Vol.-% Stickstoff zunächst 8 Stunden lang bei 140°C über den Katalysator geleitet und die Temperatur dann im Laufe von 4 Stunden auf 180°C erhöht und die Reduktion hier 4 weitere Stunden fortgesetzt. Schliesslich wird der Stickstoff durch Wasserstoff ersetzt und die Temperatur auf 300°C erhöht. Nach weiteren 8 Stunden ist die Reduktion beendet.

### Die Umsetzung

In der Apparatur wird ein Druck von 8 bar eingestellt. Mit Hilfe des Kompressors wird kontinuierlich ein Gemisch aus 50 Vol.-% Wasserstoff und 50 Vol.-% Ammoniak in einer Menge von 4 $Nm^3$ im Kreis durch die Apparatur gepumpt. Im Reaktor wird eine Temperatur von 210°C eingestellt. Sodann werden dem Verdampfer stündlich 0,2 l Diglykol zugeführt.

Das im Abscheider gesammelte Kondensat wurde gaschromatographisch analysiert und durch fraktionierende Destillation aufgearbeitet.

Nach Abzug von Ammoniak und Wasser enthält es 93 Gew.-% Morpholin, 3 Gew.-% Methylglykol und 4 Gew.-% Zwischenprodukte.

*Beispiel 2*

Es wurde der Katalysator des Beispiels 1 verwendet. Als Reaktionsapparat diente ein zylindrisches beheizbares Rohr mit 800 ml Füllvolumen. In der Katalysatorschüttung wurde eine Temperatur von 200°C eingestellt und stündlich 250 Nl Wasserstoff bei atmosphärischem Druck hindurchgeleitet. Dem vom Wasserstoff durchströmten Verdampfer wurden stündlich 90 Nl gasförmiges Ammoniak und 80 ml 1,2-Dipropylenglykol (Isomerengemisch) zugeführt.

Das Reaktionsprodukt wurde kondensiert, im Abscheider gesammelt, gaschromatographisch analysiert und fraktioniert destilliert.

Die Umwandlung war vollständig, das Reaktionsprodukt enthielt (ohne Berücksichtigung von Wasser und Ammoniak) 97% Dimethylmorpholin (Isomerengemisch) und 3% unbekannte Substanzen.

*Beispiel 3*

Es wird verfahren wie im Beispiel 1, jedoch mit dem Unterschied, dass anstelle von Diethylenglykol technisches, 96%iges 1,5-Pentandiol dem Verdampfer zugeführt wird.

Das im Abscheider gesammelte Kondensat wurde gaschromatographisch analysiert und fraktioniert destilliert.

Nach Abzug von Ammoniak und Wasser enthält es 92 Gew.-% Piperidin. Bezogen auf den Pentandiol-Anteil des Ausgangsstoffes ist das eine Ausbeute von 95%.

**Patentansprüche**

1. Verfahren zur Herstellung von cyclischen Iminen durch Umsetzung von Diolen mit Ammoniak unter hydrierenden Bedingungen und in Gegenwart eines Mischkatalysators auf der Grundlage von Kupfer auf Aluminiumoxid, dadurch gekennzeichnet, dass man die Aminierung in der Gasphase an einem Katalysator vornimmt, der neben Kupfer 2 bis 50 Gew.-%, bezogen auf Kupfer, an Nickel und/oder Kobalt enthält und dessen Träger Aluminiumoxid ist.

2. Katalysator, insbesondere zur Durchführung des Verfahrens nach Anspruch 1, dadurch gekennzeichnet, dass er neben Kupfer 2 bis 50 Gew.-%, bezogen auf Kupfer, an Nickel und/oder Kobalt enthält und dessen Träger Aluminiumoxid ist, wobei der Katalysator dadurch erhalten wurde, dass die mit Alkalicarbonat fällbaren Salze des Kupfers, Nickels bzw. Kobalts und des Aluminiums in wässriger Lösung mit Alkalicarbonat gefällt und die Fällung in üblicher Weise geformt, getrocknet und mit Wasserstoff aktiviert wurde.

3. Katalysator gemäss Anspruch 2, gekennzeichnet durch einen Gehalt an Kupfer und Nickel und/oder Kobalt von 10 bis 70 Gew.-%, wobei der Rest im wesentlichen aus Aluminiumoxid besteht und der Gehalt an Nickel bzw. Kobalt 2 bis 50 Gew.-%, bezogen auf Kupfer, beträgt.

**Claims**

1. A process for the preparation of cyclic imines by reacting a diol with ammonia under hydrogenating conditions in the presence of a mixed catalyst based on copper and nickel supported on alumina, wherein the amination is effected in the gas phase over a catalyst which in addition to copper contains from 2 to 50% by weight, based on copper, of nickel and/or cobalt, and whose carrier is alumina.

2. A catalyst particularly for carrying out the process as claimed in claim 1, which in addition to copper contains from 2 to 50% by weight, based on copper, of nickel and/or cobalt, and whose carrier is alumina, the catalyst having been obtained by precipitating the salts of copper, nickel and/or cobalt and of aluminum, capable of precipitation with an alkali metal carbonate, by means of an aqueous solution of such a carbonate and, using conventional methods, molding the precipitate, drying it and activating it with hydrogen.

3. A catalyst as claimed in claim 2, wherein the content of copper and nickel and/or cobalt is from 10 to 70% by weight, the remainder consisting essentially of alumina, and the content of nickel and/or cobalt is from 2 to 50% by weight, based on copper.

**Revendications**

1. Procédé de préparation d'imines cycliques par réaction de diols et d'ammoniac dans des conditions hydrogénantes en présence d'un catalyseur mixte au cuivre sur de l'oxyde d'aluminium, caractérisé en ce que l'amination est réalisée en phase gazeuse en présence d'un catalyseur contenant, à côté du cuivre, entre 2 et 50% du poids du cuivre de nickel et(ou) de cobalt, le support étant de l'oxyde d'aluminium.

2. Catalyseur, en particulier pour la mise en oeuvre du procédé selon la revendication 1, caractérisé en ce qu'il contient, à côté de cuivre, entre 2 et 50% du poids du cuivre de nickel et(ou) de cobalt, le support étant de l'oxyde d'aluminium, ce catalyseur étant préparé par précipitation des sels du cuivre, du nickel ou du cobalt et de l'aluminium, précipitables par les carbonates de métaux alcalins, en solution aqueuse par un carbonate de métal alcalin et formage, séchage et activation par l'hydrogène du précipité formé par des techniques usuelles.

3. Catalyseur selon la revendication 2, caractérisé en ce qu'il contient entre 10 et 70% en poids de cuivre et de nickel et(ou) de cobalt, le reste étant essentiellement constitué d'oxyde d'aluminium, sa teneur en nickel et(ou) cobalt étant de 2 à 50% du poids du cuivre.